# DEMANDE DE BREVET EUROPEEN

(11) **EP 3 799 059 A1**
(43) Date de publication de la demande: **31.03.2021**
(21) Numéro de dépôt: 20187224.9
(22) Date de dépôt: 22.07.2020
(51) Int. Cl.: G16H 20/17, G16H 50/20

(54) **DISPOSITIF POUR DÉTERMINER ET AFFICHER UNE DOSE D'INSULINE À ADMINISTRER À UN PATIENT DIABÉTIQUE**

(30) Priorité: 25.09.2019 FR 1910598
(71) Demandeur: L'AIR LIQUIDE, SOCIETE ANONYME POUR L'ETUDE ET L'EXPLOITATION DES PROCEDES GEORGES CLAUDE, 75007 Paris (FR); Alehos Development, 94250 Gentilly (FR)
(72) Inventeur: MEGHERBI, Fouad, 94250 Gentilly (FR); MOUSIN, Flavien, 94250 Gentilly (FR); CARLIER, Sébastien, 69463 Lyon Cedex 06 (FR); CRONE, Véronique, 94250 Gentilly (FR); NOEL, Nathalie, 94250 Gentilly (FR)
(74) Mandataire: Air Liquide

(57) **Abrégé**

L'invention concerne un dispositif (1) pour déterminer et afficher une dose d'insuline à administrer à un patient diabétique, que ce soit par injection, inhalation, prise orale ou autre, comprenant des moyens de fourniture de paramètres pour fournir une quantité de glucides, une quantité de fibres, une valeur d'insuline active (IOB) et une valeur de glycémie d'un patient ; des moyens de calcul (3) configurés pour calculer une dose d'insuline à partir de ces différents paramètres fournis ; et des moyens d'affichage (4) configurés pour afficher au moins la dose d'insuline calculée par les moyens de calcul (3), et/ou des moyens de mémorisation (32) configurés pour mémoriser au moins la dose d'insuline calculée par les moyens de calcul (3).

## Description

La présente invention concerne un dispositif pour déterminer et afficher une dose d'insuline à administrer, par exemple à injecter, à un patient diabétique, laquelle est calculée à partir des quantités de glucides (i.e. de composés carbohydratés ou « carbohydrates ») et de fibres présentes dans les aliments, ainsi que la valeur d'insuline active (IOB), la valeur de glycémie du patient et préférentiellement la valeur d'apport énergétique des aliments considérés.

Les millions de personnes vivant avec le diabète et traitées par insuline doivent pouvoir connaître exactement la quantité d'insuline et de bolus, c'est-à-dire combien d'unités d'insuline s'injecter en fonction de leur glycémie préprandiale, des aliments qu'ils vont consommer pendant leurs repas et de leur activité physique.

Connaître précisément la quantité d'insuline à s'injecter leur permet d'éviter des augmentations importantes des glycémies liées aux ingestions de repas, i.e. hyperglycémies, ou des baisses trop importantes des niveaux de glycémie, i.e. hypoglycémies, suite à une injection d'un bolus (i.e. quantité) d'insuline trop élevée par rapport à la quantité de glucides apportés par les repas.

La gestion de l'insulinothérapie nécessite une éducation intensive du patient et de son entourage, et obéit à des règles intégrant de nombreux facteurs, i.e. glycémie préprandiale, glucides du repas, sensibilité à l'insuline (facteur de correction), le rapport insuline/glucides qui peut être différent selon le type de repas chez une même personne diabétique...

Certaines pompes d'administration d'insuline comprennent des calculateurs de bolus intégrant également l'insuline active comme facteur de correction, c'est-à-dire en plus du calcul sur les paramètres précédemment cités.

Par exemple, EP-A-3295342 propose un dispositif de management du diabète pour déterminer un bolus d'insuline à partir d'un facteur carbohydrate (i.e. glucides) et d'une connaissance du type de diabète, i.e. type 1 ou 2, chez un patient donné. Le dispositif prend aussi en compte les niveaux d'insuline et l'activité physique du patient.

Par ailleurs, US-A-2009/0177147 propose une pompe à insuline avec des moyens de réception de données relatives au management du diabète de l'utilisateur, des boutons de sélection et un écran d'affichage. La délivrance de l'insuline se fait sur la base des informations reçues et d'une période de temps précédant un repas. La quantité d'insuline à administrer peut être calculée à partir de des différents nutriments présents dans la nourriture à ingérer, en particulier les carbohydrates, les protéines et les lipides, et optionnellement une quantité de fibres.

Toutefois, malgré ces dispositifs de calcul de bolus d'insuline, certains patients n'arrivent pas à atteindre un équilibre glycémique.

Le problème est dès lors de proposer un dispositif pour déterminer et afficher une dose d'insuline, ou bolus, plus précise que celle fournie par les dispositifs existants, c'est-à-dire de rendre le calcul de la dose d'insuline plus précis afin de mieux assister les patients sous insuline au moment de leurs repas.

La solution de l'invention concerne alors un dispositif pour déterminer et afficher une dose d'insuline (i.e. un bolus) comprenant :
- des moyens de fourniture de paramètres configurés pour fournir, c'est-à-dire entrer, sélectionner, fixer, capturer ou autre les paramètres suivants, à savoir une quantité de glucides, une quantité de fibres présentes dans des aliments, une valeur d'insuline active (IOB) et une valeur de glycémie d'un patient, lesdits moyens de fourniture de paramètre comprenant des moyens d'interface utilisateur,
- des moyens de calcul configurés pour calculer une dose d'insuline à partir d'au moins la quantité de glucides, la quantité de fibres fournies, la valeur d'insuline active (IOB) et la valeur de glycémie du patient, et
- des moyens d'affichage configurés pour afficher au moins la dose d'insuline calculée par les moyens de calcul, et
- des moyens de mémorisation configurés pour mémoriser (i.e. enregistrer) au moins la dose d'insuline calculée par les moyens de calcul,
et dans lequel :
- lesdits moyens d'interface utilisateur, lesdits moyens de calcul, lesdits moyens d'affichage et lesdits moyens de mémorisation sont embarqués dans le dispositif, et
- le dispositif est choisi parmi les téléphones mobiles multifonctions (i.e. smartphones), les tablettes numériques, les ordinateurs portables ou fixes, les montres connectées, les systèmes de mesure continue de glucose (CGM) et les dispositifs de calcul autonomes.

Dans le cadre de l'invention :
- les termes « dispositif », « appareils » et « système » sont considérés comme totalement équivalents et utilisés indifféremment.
- les quantités de glucides, de fibres ou d'autres paramètres correspondent à des valeurs évaluées, par exemple pesées, ou estimées, donc pouvant être approximatives.
- le terme « fibres » désigne des fibres alimentaires solubles et/ou insolubles, notamment d'origine végétale.
- l'insuline active ou IOB pour "Insulin On Board", est la quantité d'insuline d'action rapide injectée dans les heures, typiquement les 1 à 3 heures, précédents l'injection, généralement dans les 2 ou 3 heures, pour laquelle l'utilisateur réalise le calcul de dose. Prendre en compte l'IOB permet de minimiser le risque hypoglycémique dû à l'effet cumulatif des deux insulines.
- la glycémie du patient, c'est-à-dire la valeur de glucose sanguin avant le repas du patient. La valeur de glycémie du patient est une valeur de glycémie réelle, calculée ou prédite.

Selon le mode de réalisation considéré, le dispositif de l'invention peut comprendre l'une ou plusieurs des caractéristiques suivantes :
- les moyens de fourniture de paramètres sont configurés en outre pour fournir une valeur d'apport énergétique des aliments. Elle est généralement exprimée en Kcal ou KJoule.
- les moyens de calcul sont par ailleurs configurés pour calculer la dose d'insuline en utilisant en outre ladite valeur d'apport énergétique des aliments.
- les moyens d'interface utilisateur comprennent une ou plusieurs touches, boutons, curseurs, clavier, ou autre, par exemple un microphone d'un système à commande vocale.
- les touches, boutons, curseurs ou clavier sont mécaniques ou digitaux, par exemple un clavier ou des touches virtuelles présentes ou s'affichant sur un écran tactile.
- les moyens d'interface utilisateur comprennent un écran digital, i.e. numérique, de préférence un écran tactile.
- selon un autre mode de réalisation, les moyens d'interface utilisateur comprennent des moyens d'acquisition digitaux permettant une détermination ou une évaluation des quantités de glucides et de fibres présentes dans des aliments via une prise de photo(s) ou une capture d'image(s) suivie d'une analyse d'image/photo.
- les moyens d'acquisition digitaux comprennent un objectif numérique, tel celui d'un appareil photo, d'une caméra ou analogue, permettant de capturer une ou des images/photos des aliments à évaluer, ou de scanner/capturer un code-produit, par exemple un code à barres, un code QR ou tout autre code, se trouvant sur l'emballage d'un produit alimentaire, par exemple un plat préparé...
- les moyens d'acquisition digitaux coopèrent avec un (ou des) microprocesseur(s) mettant en œuvre un (ou plusieurs) algorithme(s) de traitement d'image configuré pour traiter informatiquement les images/photos prises par les moyens d'acquisition digitaux et en déduire une quantité de glucides et une quantité fibres.
- de façon alternative, les moyens d'acquisition digitaux coopèrent avec un (ou des) microprocesseur(s) mettant en œuvre un (ou plusieurs) algorithme(s) de traitement d'image configuré pour traiter informatiquement le code-produit ayant été scanné et en extraire une (information de) quantité de glucides et de quantité fibres.
- les moyens d'acquisition digitaux peuvent être agencés dans un appareil annexe, tel un téléphone intelligent (smartphone) à caméra embarquée, lequel coopère avec le dispositif de l'invention de manière à lui transmettre les photos/images ou codes scannées, ou bien directement intégrés au dispositif de l'invention.
- les moyens de calcul comprennent au moins un microprocesseur, de préférence au moins un microcontrôleur.
- les moyens de calcul comprennent au moins un microprocesseur mettant en œuvre un (ou des) algorithme(s).
- les moyens de calcul comprennent au moins microprocesseur agencé sur une carte électronique.
- les moyens de calcul sont en outre configurés pour calculer une dose d'insuline à partir d'une valeur d'apport énergétique, i.e. calorique, des aliments considérés (exprimée en kilocalories).
- les moyens de calcul sont en outre configurés pour calculer une dose d'insuline à partir d'au moins un paramètre additionnel choisi parmi :
   ▪ la quantité de lipides et/ou de protéines présents dans la nourriture.
   ▪ l'activité physique du patient et son intensité que l'utilisateur peut indiquer avant de commencer un exercice physique afin que la dose d'insuline soit revue en fonction de l'intensité de cet exercice physique et de la quantités de glucides et de fibres ingérées.
   ▪ une sensibilité à l'insuline du patient, c'est-à-dire l'effet d'une unité d'insuline chez un individu diabétique lors d'une épreuve de jeûn.
   ▪ un débit basal d'insuline dit aussi "insuline pour vivre". Un système délivrant de l'insuline basale peut fournir la quantité d'insuline active au dispositif de calcul.
- en particulier, les moyens de calcul sont en outre configurés pour calculer la dose d'insuline à partir d'au moins un autre paramètre additionnel choisi parmi une quantité de lipides et/ou une quantité de protéines, ou tout autre traitement pouvant modifier la cinétique de l'insuline.
- les moyens de calcul peuvent être en outre configurés pour calculer la dose d'insuline à partir en outre d'au moins une donnée physiologique du patient choisie parmi l'âge, le sexe, le stade de sa pathologie, la durée (i.e. ancienneté) de sa pathologie, le poids, la taille, l'IMC, le type de son diabète ou autre.
- les moyens de calcul peuvent être en outre configurés pour calculer la dose d'insuline à partir en outre d'au moins une caractéristique du système d'administration, notamment un type de pompe à insuline, de stylo à insuline, de système d'inhalation d'insuline ou autre.
- les moyens de calcul peuvent être en outre configurés pour calculer une dose d'insuline à partir d'au moins une caractéristique du système de mesure de la glycémie. En effet, les capteurs ou lecteurs de glycémie peuvent avoir des précisions différentes qu'il pourrait être utile, dans certains cas, de pondérer ou corriger.
- les moyens d'affichage comprennent un écran digital ou autre.
- les moyens d'affichage affichent la dose d'insuline sous forme d'une valeur numérique exprimée en unité(s) d'insuline ou en type de bolus dont la quantité a été préprogrammée.
- les moyens de mémorisation sont configurés pour assurer un stockage d'autres données, d'informations ou autres, par exemple la quantité de glucides et/ou la quantité de fibres.
- les moyens de mémorisation comprennent au moins une mémoire d'enregistrement de données.
- la mémoire est par exemple de type mémoire flash.
- la (ou les) mémoire est agencée sur la carte électronique.
- il comprend en outre une source de courant électrique, i.e. des moyens de fourniture de courant électrique.
- la source de courant électrique comprend une ou plusieurs piles ou batteries, de préférence rechargeable(s).
- la source de courant électrique alimente les moyens de calcul, les moyens d'affichage, les moyens d'interface utilisateur et/ou les moyens de mémorisation.
- les moyens de calcul et la source de courant électrique sont agencés dans un boitier, en particulier un boitier rigide, par exemple en polymère, i.e. plastique, ou en métal.
- les moyens de calcul coopèrent avec les moyens d'interface utilisateur et les moyens d'affichage.
- les moyens de calcul sont configurés pour récupérer et traiter un ou des signaux fournis par les moyens d'interface utilisateur ou par les moyens d'acquisition digitaux.
- les moyens de calcul sont configurés pour fournir au moins un signal représentatif d'une dose d'insuline (bolus) à afficher auxdits moyens d'affichage.
- il comprend des moyens de télécommunication configuré pour émettre des données (i.e. informations ou autres), notamment les valeurs saisies et/ou calculées, telles les quantités de glucides et de fibres fournies et/ou la dose d'insuline calculée, vers un serveur distant ou un ordinateur distant.
- les données comprennent les quantités de glucides et de fibres fournies, la valeur d'insuline active (IOB), la valeur de glycémie du patient, la valeur d'apport énergétique des aliments et/ou la dose d'insuline calculée.
- les moyens de télécommunication peuvent comprendre un émetteur de type modem ou analogue, une antenne émettrice ou analogue...
- les moyens de télécommunication peuvent fonctionner en mode wifi, Bluetooth, GSM, Lora, Sigfox ou autre...

D'une façon générale, le dispositif de l'invention est un dispositif autonome. Il peut être, selon le mode de réalisation désiré, choisi parmi les téléphones mobiles multifonctions (i.e. smartphones), les tablettes numériques, les ordinateurs portables ou fixes, les montres connectées, les systèmes de mesure continue de glucose (CGM) et les dispositifs de calcul autonomes, de préférence un téléphone mobile multifonction.

L'invention va maintenant être mieux comprise grâce à la description détaillée suivante, faite à titre illustratif mais non limitatif, en référence à :

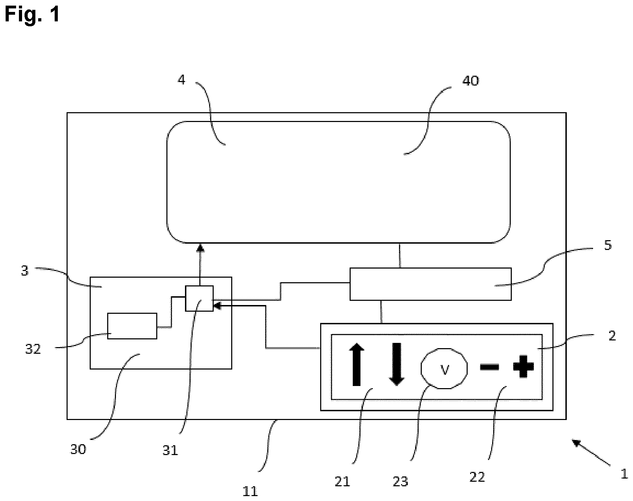

Fig. 1 qui schématise un mode de réalisation d'un dispositif autonome pour déterminer et afficher une dose d'insuline selon la présente invention.

Un dispositif 1 selon l'invention pour déterminer et afficher une dose ou bolus d'insuline, illustré en Fig. 1, comprend des moyens de fourniture de paramètres pour fournir (i.e. moyens de fourniture de quantités), c'est-à-dire entrer, sélectionner, choisir ou autre, plusieurs paramètres utiles au calcul de la dose d'insuline, i.e. bolus à administrer au patient, à savoir une quantité de glucides et une quantité de fibres correspondant aux proportions approximatives de glucides et de fibres se trouvant dans des aliments qu'une personne diabétique, c'est-à-dire l'utilisateur ou une autre personne, s'apprête ou envisage d'ingérer, i.e. de manger. Les moyens de fourniture de paramètres permettent aussi de fournir une valeur d'insuline active (IOB) et une valeur de glycémie d'un patient, voire une valeur d'apport énergétique des aliments (en KJoule ou Kcal). Ces paramètres sont utilisés pour déterminer une dose précise d'insuline à proposer au patient.

Les moyens de fourniture de paramètres peuvent comprendre des moyens d'interface utilisateur 2 permettant à un utilisateur d'entrer ou fournir les quantités de glucides et de fibres susmentionnées, ainsi que l'IOB, la valeur de glycémie et la valeur d'apport énergétique, lesquels peuvent être manuels ou automatisés, ou les deux.

Ainsi, selon un mode de réalisation, les moyens d'interface utilisateur 2 sont manuels. Ils peuvent alors comprendre des boutons, des touches, des curseurs, un clavier ou autres, qu'ils soient mécaniques ou digitaux (i.e. virtuel), par exemple des touches de défilement « haut » et « bas », comme des flèches 21, pour sélectionner le ou les paramètres à régler au sein de menus défilants, des touches « + » et « - » 22 pour incrémenter ou décrémenter des valeurs numériques, une touche de validation « V » 23 pour confirmer une sélection de paramètre ou un réglage de valeur numérique.

Selon un autre mode de réalisation, les moyens d'interface utilisateur 2 peuvent être automatisés et comprendre des moyens d'acquisition digitaux des quantités de glucides et de fibres présentes dans des aliments, par exemple une prise de photo(s) via un appareil photo, une caméra ou analogue, suivie d'une analyse d'image des aliments à évaluer/analyser par un microprocesseur ou analogue, ou, selon un autre mode de réalisation, un scannage d'un code-produit, par exemple un code à barres, un code QR code ou tout autre code, pouvant se trouver notamment sur l'emballage d'un produit alimentaire, par exemple un plat préparé. Les moyens d'acquisition digitaux peuvent être agencés dans un appareil annexe, tel un téléphone intelligent (smartphone) à caméra embarquée, ou bien intégrés au dispositif 1 de l'invention.

A noter que, selon un mode de réalisation particulier, tout ou partie des repas favoris du patient peuvent être enregistrés dans une mémoire, c'est-à-dire des valeurs préétablies notamment de quantités de glucides et de fibres, voire d'autres paramètres (e.g. IOB, glycémie, lipides, Kcal...), par exemple à partir des compositions données sur les étiquettes présentes sur les emballages des plats préparés industriels.

Le dispositif 1 comprend en outre des moyens de calcul 3, typiquement un microprocesseur 31, typiquement un microcontrôleur, porté par une carte électronique 30, qui sont configurés pour calculer une dose ou bolus d'insuline à administrer à la personne diabétique à partir d'au moins les quantités de glucides et de fibres fournies via les moyens d'interface utilisateur 2, ainsi que des valeurs d'IOB et de glycémie, voire d'apport énergétique.

L'administration de l'insuline peut se faire par injection, inhalation, prise orale ou autre, par exemple au moyen d'une pompe à insuline ou d'un stylo à insuline.

Le calcul de la dose d'insuline à afficher se fait à partir des quantités de glucides et de fibres, et des valeurs d'IOB et de glycémie, voire d'apport énergétique, fournies par l'utilisateur, lesquelles peuvent être utilisées dans une formule mathématique de calcul préétablie, dans une ou des tables ou matrices de correspondance ou autre, qui peut (peuvent) être enregistrée(s) dans la mémoire de stockage 32 d'informations et/ou de données, agencée par exemple sur la carte électronique 30 et coopérant avec le microprocesseur 31.

Différentes formules mathématiques ou tables ou matrices peuvent être utilisées pour calculer la dose d'insuline. Elles peuvent être obtenues de manière empirique via de simples essais classiques ou autres. Elles dépendent notamment du ou des autres paramètres que l'on souhaite prendre en compte dans le calcul, c'est-à-dire en plus des quantités de glucides et de fibres, des valeurs d'IOB et de glycémie, voire d'apport énergétique, selon l'invention. Dès lors, la (ou les) formule mathématique, table ou matrice proprement dite(s) utilisée(s) ne fait (font) pas partie de la présente invention.

Selon un mode de réalisation, le calcul de la dose d'insuline peut aussi être réalisé par un (ou des) algorithme(s) résultant d'un apprentissage automatique ou « machine learning » réalisé à partir d'observations postprandiales chez des patients diabétiques.

La mémoire de stockage 32, par exemple une mémoire flash, est aussi configurée pour enregistrer les valeurs de dose d'insuline ayant été calculées, ainsi que les quantités de glucides et de fibres, les valeurs d'IOB et de glycémie, voire d'apport énergétique, ayant été fournies, ainsi que toute autre information ou donnée présentant un intérêt.

Préférentiellement, après calcul, des moyens d'affichage 4, tel un écran digital 40, permettent d'afficher, c'est-à-dire de visualiser, la dose d'insuline ayant été calculée par les moyens de calcul 3 de manière à permettre à l'utilisateur de savoir quelle dose d'insuline doit être réglée sur la pompe à insuline, c'est-à-dire le bolus nécessaire pour faire face au prochain repas constitué des aliments considérés.

Alternativement, la (ou des) dose d'insuline ayant été calculée par les moyens de calcul 3 peut aussi être mémorisée par les moyens de mémorisation 32, ou même les deux, à savoir mémorisée et affichée.

Le dispositif 1 comprend en outre une source de courant électrique, c'est-à-dire des moyens de fourniture de courant électrique, par exemple une ou plusieurs piles ou batteries, de préférence rechargeable(s), qui alimente directement ou indirectement en courant électrique, les moyens de calcul, les moyens d'affichage, les moyens d'interface utilisateur et tout autre élément du dispositif 1 requérant du courant électrique pour fonctionner, notamment la carte électronique 30 et les autres composants s'y trouvant, tel que le microcontrôleur 31 et la mémoire 32.

Selon un mode de réalisation, l'ensemble des éléments constitutifs du dispositif 1 de l'invention peuvent être agencés dans un boîtier rigide, tel du polymère, i.e. plastique, du métal, de manière à constituer un dispositif autonome, typiquement un dispositif calculateur de dose d'insuline autonome.

Selon encore un autre mode de réalisation, l'ensemble des éléments constitutifs du dispositif 1 de l'invention peuvent prendre la forme d'un dispositif mobile de type téléphone mobile multifonction (smartphone), tablette numérique ou ordinateur portable, ou fixe de type ordinateur personnel (i.e. PC).

D'une façon générale, le dispositif 1 de l'invention permet de rendre le calcul de la dose d'insuline à administrer, notamment à injecter, au patient diabétique plus précis, quel que soit son mode d'administration requis en se basant sur davantage de paramètres nutritionnels, en particulier les fibres et les valeurs d'IOB et de glycémie, voire d'apport énergétique.

En effet, il a été mis en évidence dans le cadre de la présente invention que la quantité de fibres présentes dans les aliments jouent un rôle important dans le calcul de la dose d'insuline à administrer, notamment à injecter, car « contrebalançant » les effets des glucides, ce qui explique les difficultés de certains patients pour atteindre un équilibre glycémique avec les dispositifs actuels.

Plus précisément, pour une même quantité de glucides présents dans des aliments devant être ingérés par un patient diabétique, la dose d'insuline à administrer sera variable en fonction de la quantité de fibres qui s'y trouve. Plus la quantité de fibres est élevée, plus la dose d'insuline devra être modulée, i.e. réduite (pour une même quantité de glucides).

Dans le cadre de l'invention, on prend en compte les quantités de fibres et de glucides mais aussi les valeurs d'IOB et de glycémie, voire d'apport énergétique, qui sont d'autres paramètres très importants pour réaliser un calcul précis de la dose d'insuline à afficher.

On note par ailleurs que d'autres paramètres peuvent aussi influencer, dans une moindre mesure, le calcul de la valeur d'insuline, notamment les lipides, les protéines...

De là, le dispositif 1 de l'invention peut être configuré pour fournir une recommandation de dose d'insuline en prenant en compte la quantité de glucides et de fibres, comme expliqué ci-dessus, ainsi que l'apport énergétique/calorique des aliments, l'IOB et la glycémie, qu'elle soit une valeur de glycémie réelle, calculée ou prédite, mais aussi au moins un paramètre additionnel, tels les paramètres susmentionnés, tels que quantités de lipides et/ou de protéines présentes dans les aliments considérés, l'activité physique du patient et son intensité, la sensibilité à l'insuline du patient, le débit basal ou tout autre paramètre pouvant affecter le profil glycémique, tel que : aire sous courbe de la glycémie, pic glycémique (dans les 2 à 3 heures précédant le bolus à administrer), durée de l'excursion glycémique (liée à l'ingestion d'un repas ou à l'administration de l'insuline), vitesse de variation de la glycémie ou du glucose interstitiel (ou autre moyen de mesurer la glycémie i.e. cutané)...

En outre, le calcul de dose d'insuline peut aussi prendre en compte une ou des données physiologiques du patient considéré comme l'âge, le sexe, le poids, la taille, l'IMC, le stade de sa pathologie... et/ou une ou des caractéristiques du système d'injection, comme le type de pompe à insuline....

Le dispositif 1 de l'invention peut être utilisé par le patient diabétique lui-même, mais aussi par son aidant naturel (personne assistant le patient diabétique lorsque ce dernier n'est pas autonome pour s'administrer lui-même son traitement), un professionnel de santé ou toute personne habilitée à aider le patient à calculer la dose d'insuline qu'il doit s'injecter au moment d'un repas.

Il affiche ensuite sur l'écran d'affichage 40, une suggestion ou proposition de la quantité d'insuline, par exemple du nombre d'unités d'insuline, à administrer au patient. De préférence, il garde en mémoire toutes les données.

Selon un mode de réalisation particulier, le dispositif 1 de l'invention peut aussi être doté de moyens de télécommunication permettant d'émettre des données (i.e. informations ou autres), notamment les valeurs saisies et/ou calculées, telles les quantités de glucides et de fibres fournies et/ou la dose d'insuline calculée, vers un serveur distant ou analogue (i.e. ordinateur...) où ces données peuvent être stockées, analysées, traitées... afin d'assurer notamment un suivi à distance du patient.

D'une façon générale, le dispositif de l'invention permet de déterminer et d'afficher et/ou de mémoriser une dose d'insuline, ou bolus, plus précise que celle fournie par les dispositifs existants, donc de rendre le calcul de la dose d'insuline plus précis afin de mieux assister les patients sous insuline au moment de leurs repas, voire même pourrait conduire à une réduction de la dose d'insuline injectée chez certains patients.

## Revendications

1. Dispositif (1) pour déterminer et afficher une dose d'insuline comprenant :
- des moyens de fourniture de paramètres configurés pour fournir une quantité de glucides, une quantité de fibres présentes dans des aliments, une valeur d'insuline active (IOB) et une valeur de glycémie d'un patient, lesdits moyens de fourniture de paramètre comprenant des moyens d'interface utilisateur (2),
- des moyens de calcul (3) configurés pour calculer une dose d'insuline à partir d'au moins la quantité de glucides, la quantité de fibres fournies, la valeur d'insuline active (IOB) et la valeur de glycémie du patient, et
- des moyens d'affichage (4) configurés pour afficher au moins la dose d'insuline calculée par les moyens de calcul (3), et
- des moyens de mémorisation (32) configurés pour mémoriser au moins la dose d'insuline calculée par les moyens de calcul (3),
et dans lequel :
- lesdits moyens d'interface utilisateur (2), lesdits moyens de calcul (3), lesdits moyens d'affichage (4)) et lesdits moyens de mémorisation (32) sont embarqués dans le dispositif (1), et
- le dispositif (1) est choisi parmi les téléphones mobiles multifonctions (i.e. smartphones), les tablettes numériques, les ordinateurs portables ou fixes, les montres connectées, les systèmes de mesure continue de glucose (CGM) et les dispositifs de calcul autonomes.

2. Dispositif selon la revendication 1, **caractérisé en ce que** les moyens de fourniture de paramètres sont en outre configurés pour fournir une valeur d'apport énergétique des aliments, et les moyens de calcul (3) sont par ailleurs configurés pour calculer la dose d'insuline en utilisant ladite valeur d'apport énergétique des aliments.

3. Dispositif selon la revendication 1, **caractérisé en ce que** les moyens d'interface utilisateur (2) comprennent une ou plusieurs touches, boutons ou curseurs.

4. Dispositif selon la revendication 1, **caractérisé en ce que** les moyens d'interface utilisateur (2) comprennent des moyens d'acquisition digitaux, de préférence ils comprennent un objectif numérique.

5. Dispositif selon la revendication 1, **caractérisé en ce que** les moyens de calcul (3) comprennent au moins un microprocesseur (31), de préférence un microcontrôleur.

6. Dispositif selon la revendication 1, **caractérisé en ce que** les moyens d'affichage (4) comprennent un écran numérique (40).

7. Dispositif selon l'une des revendications 1 ou 5, **caractérisé en ce que** les moyens de calcul (3) comprennent au moins un microprocesseur (31) agencé sur une carte électronique (30).

8. Dispositif selon la revendication 1, **caractérisé en ce qu'**il comprend en outre une source de courant électrique (5), notamment une ou plusieurs piles ou batteries.

9. Dispositif selon l'une des revendications 1, 5 ou 7, **caractérisé en ce que** les moyens de calcul (3) sont en outre configurés pour calculer la dose d'insuline à partir d'au moins un paramètre additionnel choisi parmi une quantité de lipides et/ou une quantité de protéines.

10. Dispositif selon la revendication 1, **caractérisé en ce qu'**il est choisi parmi les téléphones mobiles multifonctions, les tablettes numériques, et les montres connectées.

11. Dispositif selon la revendication 1 ou 6, **caractérisé en ce que** l'écran numérique (40) est un écran tactile.

12. Dispositif selon la revendication 1, **caractérisé en ce que** les moyens de calcul sont configurés pour fournir au moins un signal représentatif d'une dose d'insuline à afficher auxdits moyens d'affichage.

13. Dispositif selon la revendication 1, **caractérisé en ce qu'**il comprend des moyens de télécommunication configuré pour émettre des données choisies parmi les valeurs saisies et/ou calculées, vers un serveur distant ou un ordinateur distant.

14. Dispositif selon la revendication 13, **caractérisé en ce que** les données comprennent les quantités de glucides et de fibres fournies, la valeur d'insuline active (IOB), la valeur de glycémie du patient, la valeur d'apport énergétique des aliments et/ou la dose d'insuline calculée.
